# EUROPEAN PATENT APPLICATION

(11) **EP 4 681 649 A1**
(43) Date of publication of application: **21.01.2026**
(21) Application number: 24189396.5
(22) Date of filing: 18.07.2024
(51) Int. Cl.: A61B 6/12, A61B 6/46, A61B 6/00, A61B 6/50, A61B 8/12, A61B 18/14

(54) **RENAL NERVE BUNDLE CO-REGISTRATION WITH X-RAY IMAGE FOR RENAL DENERVATION TREATMENT GUIDANCE**

(71) Applicant: Koninklijke Philips N.V., 5656 AG Eindhoven (NL)
(72) Inventor: CEZO, James David, Eindhoven (NL); CHEN, Sara Rose, Eindhoven (NL)
(74) Representative: Philips Intellectual Property & Standards

(57) **Abstract**

An apparatus includes a processor configured for communication with an intravascular imaging catheter. The processor is configured to control the intravascular imaging catheter to obtain a plurality of intravascular images during movement of the catheter along a length of a renal artery, where the plurality of intravascular images depicts one or more nerve bundles for targeting during a renal denervation treatment. The processor performs co-registration between a plurality of nerve bundle data points representative of the nerve bundles and associated with a treatment location along the length of the renal artery for the renal denervation treatment; and a first X-ray image of the renal artery obtained with a contrast fluid inside the renal artery. The processor outputs, to a display, a screen display based on the co-registration and configured to guide a user regarding the renal denervation treatment.

## Description

### FIELD

The subject matter described herein relates to systems, devices, and methods for co-registration of renal angiography with renal nerve bundle data from intravascular imaging to provide guidance for a renal denervation (RDN) procedure. This RDN nerve data co-registration system has particular but not exclusive utility for improving speed and outcomes for renal denervation procedures.

### BACKGROUND

Renal denervation (RDN) is a potentially promising procedure for the treatment of hypertension that cannot adequately be treated with anti-hypertensive medications. Studies demonstrate effectiveness of the treatment with a response rate of about 2/3 of patients gaining a decrease of systolic blood pressure of 5 mm Hg or more. However, studies have also shown varying levels of efficacy, with some portion of the population (currently estimated at approximately 30%) showing no response or negative response to the procedure. This demographic of non-responders may present as such for reasons that may stem from a combination of physiological factors and/or inadequately administered therapy.

Currently, renal denervation procedures are performed intravascularly under fluoroscopic guidance. There is little information about the correct locations to perform the RDN procedure or the characteristics and morphology of the tissues adjacent to the artery. With fluoroscopy the user only can identify the borders of the artery in a 2D projection.

The information included in this Background section of the specification, including any references cited herein and any description or discussion thereof, is included for technical reference purposes only and is not to be regarded as subject matter by which the scope of the disclosure is to be bound.

### SUMMARY

Disclosed is an RDN nerve data co-registration system which enables a clinician to predict which treatment locations are likely to show favorable response to RDN treatment, and/or to determine the success or completion of an RDN treatment in progress. The present disclosure involves projecting co-registered data about the size and positioning of renal nerves at different locations onto fluoroscopic images of the renal artery during an RDN procedure. Currently, renal denervation (RDN) procedures are performed intravascularly under fluoroscopic guidance, with little information available about the correct locations to perform the RDN procedure or the tissues adjacent to the artery. Intravascular imaging or pre-procedural CT imaging can better assess and identify the tissues adjacent to the artery, and particularly the sizes and locations of the renal nerve bundles targeted by the RDN procedure. The presence of certain features (e.g., nerve bundles located close to the renal artery) and the absence of other features (e.g., calcifications or other features blocking access to the nerve bundles) may impact the efficacy of RDN procedures and can thus help guide the user where they should or should not use the RDN treatment device(s). The RDN nerve data co-registration system disclosed herein has particular, but not exclusive, utility for determining the suitability of locations for, and/or the completion/success of, renal denervation procedures.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the RDN nerve data co-registration system, as defined in the claims, is provided in the following written description of various aspects of the disclosure and illustrated in the accompanying drawings.

### BRIEF DESCRIPTION OF THE DRAWINGS

Illustrative aspects of the present disclosure will be described with reference to the accompanying drawings, of which:
**Figure 1** is a diagrammatic schematic view of an intraluminal imaging system, according to aspects of the present disclosure.
**Figure 2** is a schematic diagram of a processor circuit, according to aspects of the present disclosure.
**Figure 3** is a schematic, diagrammatic representation, of an intraluminal imaging catheter being pulled back through the left renal artery of the left kidney, according to aspects of the present disclosure.
**Figure 4** is a schematic, diagrammatic representation of an X-ray image, with contrast, of the renal arterial tree, according to aspects of the present disclosure.
**Figure 5** is a representation of a lateral cross-sectional IVUS image of a renal artery at a first location, according to aspects of the present disclosure.
**Figure 6** is a representation of a lateral cross-sectional IVUS image of a renal artery at a second location, different from the first location, according to aspects of the present disclosure.
**Figure 7A** is a schematic, diagrammatic representation of the co-registration of renal nerve data points with the angiographic roadmap image of the renal arterial tree, according to aspects of the present disclosure.
**Figure 7B** is a is a schematic, diagrammatic representation of the co-registration of intravascular image frames with the angiographic roadmap image of the renal arterial tree, according to aspects of the present disclosure.
**Figure 8** is a co-registration screen display of an example RDN nerve data co-registration system, according to aspects of the present disclosure.
**Figure 9** is a roadmap image screen display for an example RDN nerve data co-registration system, according to aspects of the present disclosure.
**Figure 10** is a roadmap image screen display for an example RDN nerve data co-registration system, according to aspects of the present disclosure.
**Figure 11** is a roadmap image screen display for an example RDN nerve data co-registration system, according to aspects of the present disclosure.
**Figure 12** is a schematic, diagrammatic representation, in flow diagram form, of an example RDN nerve data co-registration method, according to aspects of the present disclosure.
**Figure 13** is a schematic, diagrammatic representation, in block diagram form, of an example machine learning model training process for identifying RDN treatment locations, according to aspects of the present disclosure.
**Figure 14** is a schematic, diagrammatic representation, in block diagram form, of an example machine learning model training system for RDN treatment location calculation, according to aspects of the present disclosure.
**Figure 15** is a schematic, diagrammatic representation, in block diagram form, of an example machine learning model inference mode for RDN treatment location calculation, according to aspects of the present disclosure.
**Figure 16** is a schematic, diagrammatic representation, in flow diagram form, of an example RDN treatment evaluation process, according to aspects of the present disclosure.
**Figure 17** is a schematic, diagrammatic representation of the renal vasculature of a patient, with a renal denervation treatment device in the left renal artery of the left kidney, according to aspects of the present disclosure.

### DETAILED DESCRIPTION

In accordance with at least one aspect of the present disclosure, an RDN nerve data co-registration system is provided which enables a clinician to predict which treatment locations are likely to show favorable response to RDN treatment, and/or to determine the success or completion of an RDN treatment in progress.

The present disclosure involves projecting co-registered data onto fluoroscopic images of the renal nerve (e.g., data overlaid onto the location where the data was captured) during an RDN procedure. Currently, renal denervation (RDN) procedures are performed intravascularly under fluoroscopic guidance. There is little information available about the correct locations to perform the RDN procedure, or the characteristics and morphology of the tissues adjacent to the artery. With fluoroscopy, the user can only identify the borders of the artery in a 2D projection. Intravascular imaging or pre-procedural CT imaging can better assess and identify the tissues adjacent to the artery, such as the renal nerve, renal vein, arterial wall thickening, fatty deposits adjacent to the artery, or calcifications. The presence or absence of these features may impact the efficacy of RDN procedures, and can thus help guide the clinician as to where they should or should perform denervation using the RDN treatment device(s).

The present disclosure brings advanced imaging information onto the fluoroscopic or angiographic view that the physician is using to guide an RDN procedure. The location of the renal nerve in relation to the renal artery can aid the clinician performing the RDN to better locate their treatment devices for a successful nerve ablation.

The RDN nerve data co-registration system includes the fluoroscopic or angiographic view of the renal arterial tree taken during the procedure. Adjacent to this image would be advanced imaging such as optical coherence tomography (OCT) or intravascular ultrasound (IVUS) imaging taken from a catheter within the renal artery or computer-aided tomography (CT) imaging taken prior to the procedure. These images can contain more detailed information about the tissues surrounding the renal arterial tree.

In an aspect, the user would be able to scroll along the renal artery in the angiographic view to show the corresponding cross-sectional view of the advanced imaging.

In an aspect, this advanced imaging is generated using intravascular ultrasound (IVUS) or intravascular optical coherence tomography (OCT).

In another aspect, a computer vision system is be trained to identify structures within the advanced imaging to further guide the user to the morphology of tissues surrounding the renal artery. Some such features include the renal nerve(s), arterial wall, renal vein, fatty tissues, scar tissue, calcification, arterial wall thickening, and arterial spasm.

Additionally, an aspect would indicate the location for RDN treatment which corresponds with the best chance of a successful procedure. This location may be derived from training an algorithm on previously performed RDN procedures and correlating treatment and nerve position to clinical efficacy of the technique.

In another aspect, the advanced imaging such as IVUS is done after the RDN treatment to identify whether the nerve tissue has been appropriately ablated. IVUS imaging showing healthy and ablated nerve tissues can be co-registered with the renal artery angiography.

The present disclosure aids substantially in performing interventional procedures such as renal denervation, by improving the clinician's ability to understand where the treatment will be most effective. Implemented on a processor in communication with one or more sensors, the RDN nerve data co-registration system disclosed herein provides practical detection and measurement of the patient's renal nerves, their proximity to the renal artery, and their likely response to RDN treatment. This improved situational awareness transforms a blind medical procedure with a 70% success rate into one where the success can be accurately predicted and even measured in real time, to determine if more treatment is necessary, without the normally routine need to wait and see whether the patient's hypertension declines over a period of days or weeks. This unconventional approach improves the functioning of the renal denervation system, by improving the success rate of RDN treatments and by providing information necessary to perform the procedure only in locations where the renal nerve bundles are likely to be affected.

The RDN nerve data co-registration system may be implemented at least partially as a process viewable on a display, and operated by a control process executing on a processor that accepts user inputs from a keyboard, mouse, or touchscreen interface, and that is in communication with one or more sensors. In that regard, the control process performs certain specific operations in response to different inputs or selections made at different times. Certain outputs of the RDN nerve data co-registration system may be printed, shown on a display, indicated with lights or tones, or otherwise communicated to human operators. Certain structures, functions, and operations of the processor, display, sensors, and user input systems are known in the art, while others are recited herein to enable novel features or aspects of the present disclosure with particularity.

The present disclosure is related to U.S. Provisional Application No. ____, filed ____, and titled "Catheter-Based Procedures With Procedure Room Detection Of Biomarkers In Patient Blood And Associated Devices, Systems, And Methods" (Atty. Dkt. No. 2023PF00849 / 44755.2399PV01), U.S. Provisional Application No. ____, filed ____, and titled "Multi-Factor Renal Denervation Index For Patient Suitability and/or Expected Responsiveness To Renal Denervation Treatment" (Atty. Dkt. No. 2023PF00861 / 44755.2401PV01), U.S. Provisional Application No. ___, filed ___, and titled "Renal Denervation Treatment Guidance Using Hemodynamic Co-Registration and Associated Systems, Devices, and Methods" (Atty. Dkt. No. 2023PF00857 / 44755.2405PV01), and U.S. Provisional Application No. ___, filed ____, and titled "Renal Denervation Treatment Assessment Using Ambulatory Blood Pressure Monitor" (Atty. Dkt. No. 2024PF00043 / 44755.2414PV01), each of which is incorporated by reference as though fully set forth herein.

These descriptions are provided for exemplary purposes only, and should not be considered to limit the scope of the RDN nerve data co-registration system. Certain features may be added, removed, or modified without departing from the spirit of the claimed subject matter.

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

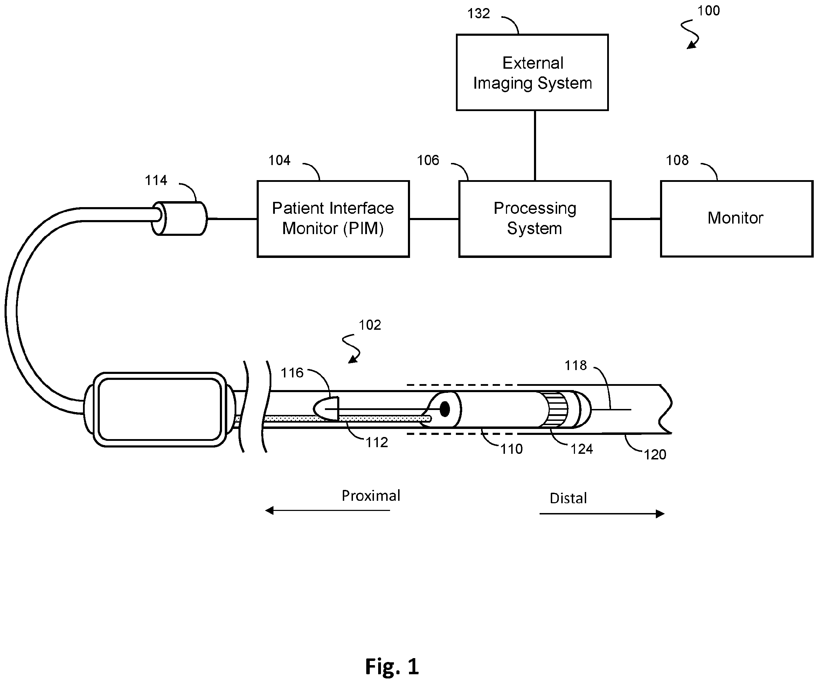

**Figure 1** is a diagrammatic schematic view of an intraluminal imaging system, according to aspects of the present disclosure. The intraluminal imaging system 100 can be an intravascular ultrasound (IVUS) imaging system in some aspects. The intraluminal imaging system 100 may include an intraluminal device 102, a patient interface module (PIM) 104, a console or processing system 106, a monitor 108, and an external imaging system 132 which may include angiography, ultrasound, X-ray, computed tomography (CT), magnetic resonance imaging (MRI), or other imaging technologies, equipment, and methods. The intraluminal device 102 is sized and shaped, and/or otherwise structurally arranged to be positioned within a body lumen of a patient. For example, the intraluminal device 102 can be a catheter, guide wire, guide catheter, pressure wire, and/or flow wire in various aspects. In some circumstances, the system 100 may include additional elements and/or may be implemented without one or more of the elements illustrated in Figure 1. For example, the system 100 may omit the external imaging system 132.

The intraluminal imaging system 100 (or intravascular imaging system) can be any type of imaging system suitable for use in the lumens or vasculature of a patient. In some aspects, the intraluminal imaging system 100 is an intravascular ultrasound (IVUS) imaging system. In other aspects, the intraluminal imaging system 100 may include systems configured for forward looking intravascular ultrasound (FL-IVUS) imaging, intravascular photoacoustic (IVPA) imaging, intracardiac echocardiography (ICE), transesophageal echocardiography (TEE), and/or other suitable imaging modalities.

It is understood that the system 100 and/or device 102 can be configured to obtain any suitable intraluminal imaging data. In some aspects, the device 102 may include an imaging component of any suitable imaging modality, such as optical imaging, optical coherence tomography (OCT), etc. In some aspects, the device 102 may include any suitable non-imaging component, including a pressure sensor, a flow sensor, a temperature sensor, an optical fiber, a reflector, a mirror, a prism, an ablation element, a radio frequency (RF) electrode, a conductor, or combinations thereof. Generally, the device 102 can include an imaging element to obtain intraluminal imaging data associated with the lumen 120. The device 102 may be sized and shaped (and/or configured) for insertion into a vessel or lumen 120 of the patient.

The system 100 may be deployed in a catheterization laboratory having a control room. The processing system 106 may be located in the control room. Optionally, the processing system 106 may be located elsewhere, such as in the catheterization laboratory itself. The catheterization laboratory may include a sterile field while its associated control room may or may not be sterile depending on the procedure to be performed and/or on the health care facility. The catheterization laboratory and control room may be used to perform any number of medical imaging procedures such as angiography, fluoroscopy, CT, IVUS, virtual histology (VH), forward looking IVUS (FL-IVUS), intraluminal photoacoustic (IVPA) imaging, a fractional flow reserve (FFR) determination, a coronary flow reserve (CFR) determination, optical coherence tomography (OCT), computed tomography, intracardiac echocardiography (ICE), forward-looking ICE (FLICE), intraluminal palpography, transesophageal ultrasound, fluoroscopy, and other medical imaging modalities, or combinations thereof. In some aspects, device 102 may be controlled from a remote location such as the control room, such than an operator is not required to be in close proximity to the patient.

The intraluminal device 102, PIM 104, monitor 108, and external imaging system 132 may be communicatively coupled directly or indirectly to the processing system 106. These elements may be communicatively coupled to the medical processing system 106 via a wired connection such as a standard copper link or a fiber optic link and/or via wireless connections using IEEE 802.11 Wi-Fi standards, Ultra Wide-Band (UWB) standards, wireless FireWire, wireless USB, or another high-speed wireless networking standard. The processing system 106 may be communicatively coupled to one or more data networks, e.g., a TCP/IP-based local area network (LAN). In other aspects, different protocols may be utilized such as Synchronous Optical Networking (SONET). In some cases, the processing system 106 may be communicatively coupled to a wide area network (WAN). The processing system 106 may utilize network connectivity to access various resources. For example, the processing system 106 may communicate with a Digital Imaging and Communications in Medicine (DICOM) system, a Picture Archiving and Communication System (PACS), and/or a Hospital Information System (HIS) via a network connection.

At a high level, an ultrasound imaging intraluminal device 102 emits ultrasonic energy from a transducer array 124 included in scanner assembly 110 mounted near a distal end of the intraluminal device 102. The ultrasonic energy is reflected by tissue structures in the medium (such as a lumen 120) surrounding the scanner assembly 110, and the ultrasound echo signals are received by the transducer array 124. The scanner assembly 110 generates electrical signal(s) representative of the ultrasound echoes. The scanner assembly 110 can include one or more single ultrasound transducers and/or a transducer array 124 in any suitable configuration, such as a planar array, a curved array, a circumferential array, an annular array, etc. For example, the scanner assembly 110 can be a one-dimensional array or a two-dimensional array in some instances. In some instances, the scanner assembly 110 can be a rotational ultrasound device. The active area of the scanner assembly 110 can include one or more transducer materials and/or one or more segments of ultrasound elements (e.g., one or more rows, one or more columns, and/or one or more orientations) that can be uniformly or independently controlled and activated. The active area of the scanner assembly 110 can be patterned or structured in various basic or complex geometries. The scanner assembly 110 can be disposed in a side-looking orientation (e.g., ultrasonic energy emitted perpendicular and/or orthogonal to the longitudinal axis of the intraluminal device 102) and/or a forward-looking looking orientation (e.g., ultrasonic energy emitted parallel to and/or along the longitudinal axis). In some instances, the scanner assembly 110 is structurally arranged to emit and/or receive ultrasonic energy at an oblique angle relative to the longitudinal axis, in a proximal or distal direction. In some aspects, ultrasonic energy emission can be electronically steered by selective triggering of one or more transducer elements of the scanner assembly 110.

The ultrasound transducer(s) of the scanner assembly 110 can be a piezoelectric micromachined ultrasound transducer (PMUT), capacitive micromachined ultrasonic transducer (CMUT), single crystal, lead zirconate titanate (PZT), PZT composite, other suitable transducer type, and/or combinations thereof. In an aspect the ultrasound transducer array 124 can include any suitable number of individual transducer elements or acoustic elements between 1 acoustic element and 1000 acoustic elements, including values such as 2 acoustic elements, 4 acoustic elements, 36 acoustic elements, 64 acoustic elements, 128 acoustic elements, 500 acoustic elements, 812 acoustic elements, and/or other values both larger and smaller.

The PIM 104 transfers the received echo signals to the processing system 106 where the ultrasound image (including the flow information) is reconstructed and displayed on the monitor 108. The console or processing system 106 can include a processor and a memory. The processing system 106 may be operable to facilitate the features of the intraluminal imaging system 100 described herein. For example, the processor can execute computer readable instructions stored on the non-transitory tangible computer readable medium.

The PIM 104 facilitates communication of signals between the processing system 106 and the scanner assembly 110 included in the intraluminal device 102. This communication may include providing commands to integrated circuit controller chip(s) within the intraluminal device 102, selecting particular element(s) on the transducer array 124 to be used for transmit and receive, providing the transmit trigger signals to the integrated circuit controller chip(s) to activate the transmitter circuitry to generate an electrical pulse to excite the selected transducer array element(s), and/or accepting amplified echo signals received from the selected transducer array element(s) via amplifiers included on the integrated circuit controller chip(s). In some aspects, the PIM 104 performs preliminary processing of the echo data prior to relaying the data to the processing system 106. In examples of such aspects, the PIM 104 performs amplification, filtering, and/or aggregating of the data. In an aspect, the PIM 104 also supplies high- and low-voltage DC power to support operation of the intraluminal device 102 including circuitry within the scanner assembly 110.

The processing system 106 receives echo data from the scanner assembly 110 by way of the PIM 104 and processes the data to reconstruct an image of the tissue structures in the medium surrounding the scanner assembly 110. Generally, the device 102 can be utilized within any suitable anatomy and/or body lumen of the patient. The processing system 106 outputs image data such that an image of the vessel or lumen 120, such as a cross-sectional IVUS image of the lumen 120, is displayed on the monitor 108. Lumen 120 may represent fluid filled or fluid-surrounded structures, both natural and man-made. Lumen 120 may be within a body of a patient. Lumen 120 may be a blood vessel, such as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

The controller or processing system 106 may include a processing circuit having one or more processors in communication with memory and/or other suitable tangible computer readable storage media. The controller or processing system 106 may be configured to carry out one or more aspects of the present disclosure. In some aspects, the processing system 106 and the monitor 108 are separate components. In other aspects, the processing system 106 and the monitor 108 are integrated in a single component. For example, the system 100 can include a touch screen device, including a housing having a touch screen display and a processor. The system 100 can include any suitable input device, such as a touch sensitive pad or touch screen display, keyboard/mouse, joystick, button, etc., for a user to select options shown on the monitor 108. The processing system 106, the monitor 108, the input device, and/or combinations thereof can be referenced as a controller of the system 100. The controller can be in communication with the device 102, the PIM 104, the processing system 106, the monitor 108, the input device, and/or other components of the system 100.

In some aspects, the intraluminal device 102 includes some features similar to traditional solid-state IVUS catheters, such those disclosed in U.S. Patent No. 7,846,101, hereby incorporated by reference in its entirety. For example, the intraluminal device 102 may include the scanner assembly 110 near a distal end of the intraluminal device 102 and a transmission line bundle 112 extending along the longitudinal body of the intraluminal device 102. The cable or transmission line bundle 112 can include a plurality of conductors, including one, two, three, four, five, six, seven, or more conductors.

The transmission line bundle 112 terminates in a PIM connector 114 at a proximal end of the intraluminal device 102. The PIM connector 114 electrically couples the transmission line bundle 112 to the PIM 104 and physically couples the intraluminal device 102 to the PIM 104. In an aspect, the intraluminal device 102 further includes a guidewire exit port 116. Accordingly, in some instances the intraluminal device 102 is a rapid- exchange catheter. The guidewire exit port 116 allows a guidewire 118 to be inserted towards the distal end in order to direct the intraluminal device 102 through the lumen 120.

The monitor 108 may be a display device such as a computer monitor or other type of screen. The monitor 108 may be used to display selectable prompts, instructions, and visualizations of imaging data to a user. In some aspects, the monitor 108 may be used to provide a procedure-specific workflow to a user to complete an intraluminal imaging procedure. This workflow may include performing a pre-stent plan to determine the state of a lumen and potential for a stent, as well as a post-stent inspection to determine the status of a stent that has been positioned in a lumen.

The external imaging system 132 can be configured to obtain X-ray, radiographic, angiographic/venographic (e.g., with contrast), and/or fluoroscopic (e.g., without contrast) images of the body of a patient (including the vessel 120). External imaging system 132 may also be configured to obtain computed tomography images of the body of the patient (including the vessel 120). The external imaging system 132 may include an external ultrasound probe configured to obtain ultrasound images of the body of the patient (including the vessel 120) while positioned outside the body. In some aspects, the system 100 includes other imaging modality systems (e.g., MRI) to obtain images of the body of the patient (including the vessel 120). The processing system 106 can utilize the images of the body of the patient in conjunction with the intraluminal images obtained by the intraluminal device 102.

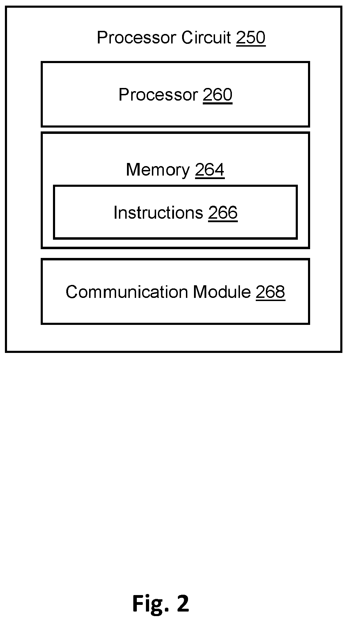

**Figure** 2 is a schematic diagram of a processor circuit 250, according to aspects of the present disclosure. The processor circuit 250 may be implemented in the intraluminal imaging system 100, or other devices or workstations (e.g., third-party workstations, network routers, etc.), or on a cloud processor or other remote processing unit, as necessary to implement the method. As shown, the processor circuit 250 may include a processor 260, a memory 264, and a communication module 268. These elements may be in direct or indirect communication with each other, for example via one or more buses.

The processor 260 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 260 may also comprise another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an aspect, the memory 264 includes a non-transitory computer-readable medium. The memory 264 may store instructions 266. The instructions 266 may include instructions that, when executed by the processor 260, cause the processor 260 to perform the operations described herein. Instructions 266 may also be referred to as code. The terms "instructions" and "code" should be interpreted broadly to include any type of computer-readable statement(s). For example, the terms "instructions" and "code" may refer to one or more programs, routines, subroutines, functions, procedures, etc. "Instructions" and "code" may include a single computer-readable statement or many computer-readable statements.

The communication module 268 can include any electronic circuitry and/or logic circuitry to facilitate direct or indirect communication of data between the processor circuit 250, and other processors or devices. In that regard, the communication module 268 can be an input/output (I/O) device. In some instances, the communication module 268 facilitates direct or indirect communication between various elements of the processor circuit 250 and/or the intraluminal imaging system 100. The communication module 268 may communicate within the processor circuit 250 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MII,-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the annular ultrasound imaging array) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles), 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

It will also be understood that one or more of the steps of the methods described above can be performed by one or more components of an ultrasound imaging system, such as the processing system, a multiplexer, a beamformer, a signal processing unit, an image processing unit, or any other suitable component of the system. For example, activating the scan sequences may be carried out by a processor in communication with a multiplexer configured to select or activate one or more elements of an ultrasound transducer array. In some aspects, generating the ultrasound images may include beamforming incoming signals from the ultrasound imaging device and processing the beamformed signals by an image processor. The processing components of the system can be integrated within the ultrasound imaging device, contained within an external console, or may be a separate component.

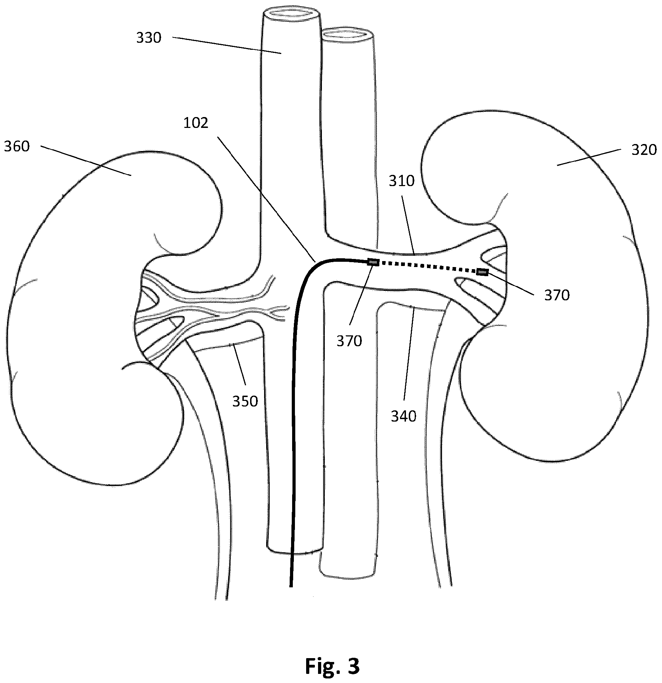

**Figure 3** is a schematic, diagrammatic representation, of an intraluminal imaging catheter 102 being pulled back through the left renal artery 310 of the left kidney 320, according to aspects of the present disclosure. The imaging component 370 (e.g., an OCT imaging element or ultrasound transducer array) intraluminal imaging catheter 102 can gather image data of the structures outside of the renal artery 310, thus providing information about the location of the renal nerves at various points along the length of the renal artery 310, as well as other structures (e.g., lesions, fatty deposits, calcifications, etc.) that may block or otherwise interfere with the delivery of treatment energy to the renal nerves.

Also visible are the abdominal aorta 330, left renal vein 340, right renal vein 350, and right kidney 360.

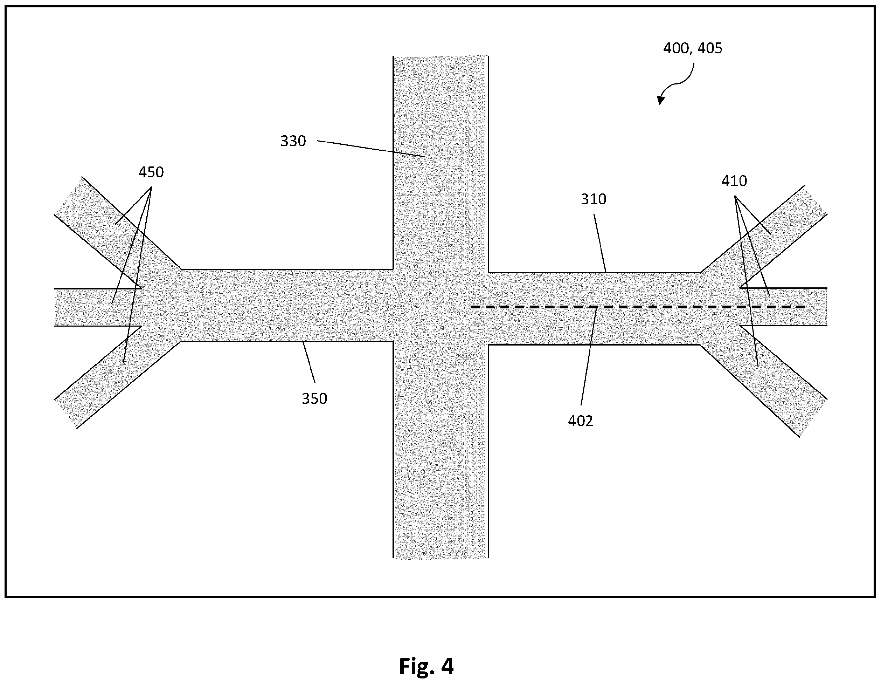

**Figure 4** is a schematic, diagrammatic representation of an X-ray image 400, with contrast, of the renal arterial tree 405, according to aspects of the present disclosure. Visible are the abdominal aorta 330, left renal artery 310, left renal artery branches 410, right renal artery 350, and right renal artery branches 450. An X-ray image with contrast may for example be taken prior to the RDN procedure to provide a roadmap of the renal arterial tree 405 to help guide the procedure. Overlaid on the renal arterial tree 405 is the pullback path 402 of the intravascular imaging device 102 of Figure 3. Figure 4 may for example be a screen display showing the renal arterial tree as a roadmap image.

In some aspects, instead of being based on an angiographic image, the roadmap image bay be a 3D model of the renal arterial tree, or a portion thereof, constructed from CT image data.

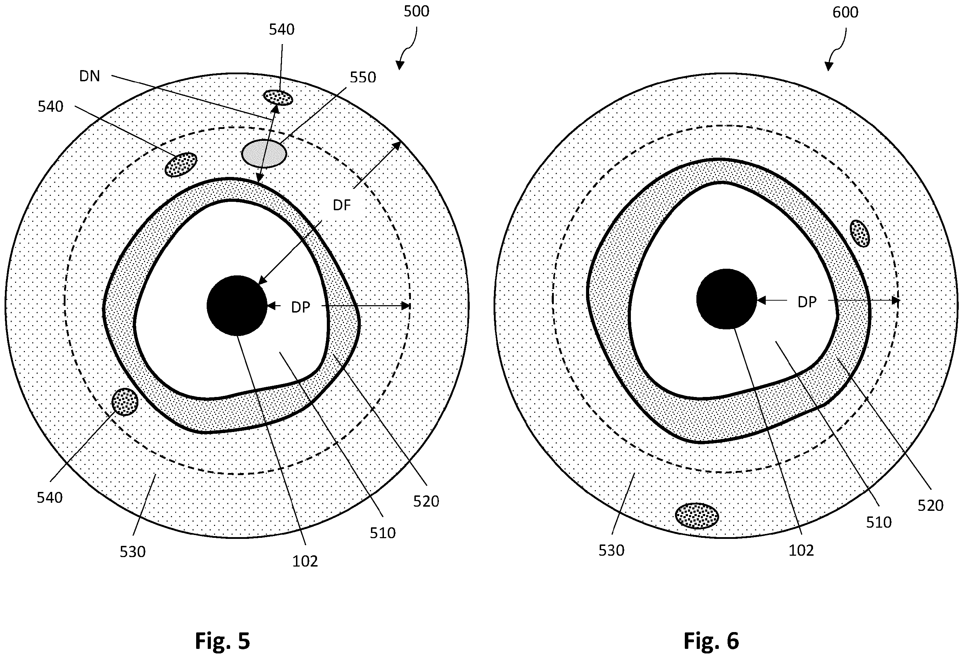

**Figure 5** is a representation of a lateral cross-sectional IVUS image 500 of a renal artery at a first location, according to aspects of the present disclosure. Visible are the imaging catheter 102, artery lumen 510, artery wall 520, surrounding tissue 530, renal nerve bundles 540, and a calcification or other obstacle 550. A first arrow DN shows the distance from the artery wall 520 to the most distant nerve bundle 540. A second arrow DP shows the depth of penetration of the renal denervation treatment (e.g., a radius of 5 mm from the center of the lumen). A third arrow DF shows the depth of field of the IVUS imaging device 102 (e.g., a radius of 10 mm from the radial center of the imaging device 102).

In the example shown in Figure 5, one nerve bundle 540 is located outside the depth of treatment penetration DP, and is also blocked by a calcification or other obstacle 550, and is thus unlikely to be affected by RDN treatment. However, two nerve bundles 540 are located within the depth of treatment penetration DP, and are not blocked by obstacles, and are thus likely to be affected by the RDN treatment. Based on this information, a clinician may decide whether this location is suitable for RDN treatment.

The identification of nerve bundles and/or the degree of ablation of the nerve bundle in intravascular images is described, for example, in U.S. Patent No. 9,615,878, filed December 20, 2013, incorporated by reference as though fully set forth herein.

**Figure 6** is a representation of a lateral cross-sectional IVUS image 600 of a renal artery at a second location, different from the first location, according to aspects of the present disclosure. Visible are the imaging catheter 102, artery lumen 510, artery wall 520, surrounding tissue 530, and renal nerve bundles 540. In the example shown in Figure 6, one nerve bundle 540 is located outside the depth of treatment penetration DP, and is thus unlikely to be affected by RDN treatment. However, one nerve bundle 540 is located within the depth of treatment penetration DP, and is not blocked by obstacles, and is thus likely to be affected by the RDN treatment. Based on this information, a clinician may decide whether this location is suitable for RDN treatment.

In some examples, without limitation, a clinician may seek a treatment location where all of the nerve bundles are located within the depth of treatment penetration. In other examples, a clinician may seek a treatment location where at least one nerve bundle is located within the depth of treatment penetration. In still other examples, the clinician may select treatment locations where a majority of the nerve bundles are located within the depth of treatment penetration. Thus, the RDN nerve data co-registration system may calculate data points with a metric for each image frame or location. The metric may for example be or include an average depth of nerve bundles, a minimum depth of nerve bundles, a maximum depth of nerve bundles, a quantity of nerve bundles visible in the frame, a size of one or more nerve bundles in the image frame, a quantity of nerve bundles located within the depth of treatment penetration, a number of nerve bundles weighted by their proximity to the wall of the renal artery, a distance between a border of the renal artery and the one or more nerve bundles, , total nerve bundle area within the depth of treatment penetration, average size of nerve bundles, density of nerve bundles within a frame, density of nerve bundles within the depth of treatment penetration, volume of nerve tissue over the length of imaging pullback, or other metrics as would occur to a person of ordinary skill in the art. The metric(s) can be used to help the clinician evaluate the RDN treatability of each location associated with an intravascular image frame. Such metrics can also be derived from a 3D model based on CT image data rather than intravascular (e.g., IVUS or OCT) image data, and associated with particular locations along the renal artery where the metric was calculated. In either case, the metric is representative of a structure of one or more nerve bundles, and of a geometry, a structural, spatial, or geometrical relationship between the nerve bundles and the renal artery wall.

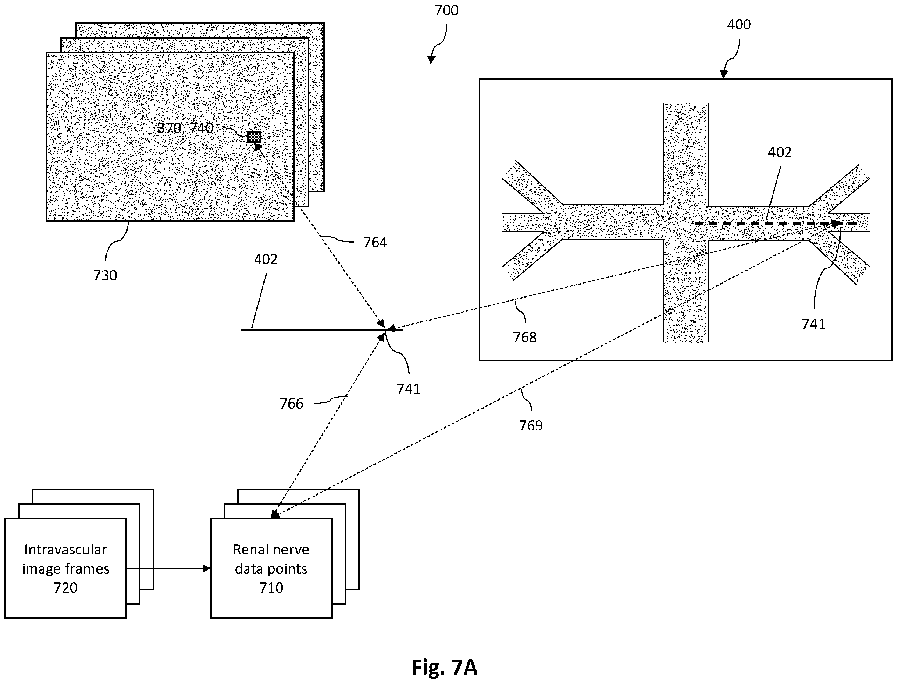

**Figure 7A** is a schematic, diagrammatic representation 700 of the co-registration of renal nerve data points 710 with the angiographic roadmap image 400 of the renal arterial tree, according to aspects of the present disclosure. In the example shown in Figure 7A, the location of the imaging element 370 or RDN treatment device 740 is visible in frames 730 of a live fluoroscopic X-ray display taken during the RDN procedure. Each such location is correlated with a renal nerve data point 710, derived from the intravascular image frames 720 along the pullback path 402, such that renal nerve data points 710 can be added (e.g., as visual annotations) to locations of intravascular image frames 720 on the pullback path 402 of the roadmap, along with the current location of the imaging array 370 or RDN treatment device 740.

As shown by the arrow 764, because the pullback path 402 is correlated with the fluoroscopy images 730, each location 741 along the path 402 may be associated with one or more fluoroscopy images 730. In addition, because a correspondence was also established between the fluoroscopy images 730 and the intravascular renal nerve data points 710, the data points 710 may also be associated with the location 741 along the path 402 as shown by the arrow 766. Just as different fluoroscopy images 730 and intravascular renal nerve data points 710 were associated with various locations along the pullback path 402, the fluoroscopy images 730 and intravascular renal nerve data points 710 may be associated with the same locations along the roadmap image 400, as shown by the arrow 768 and the arrow 769.

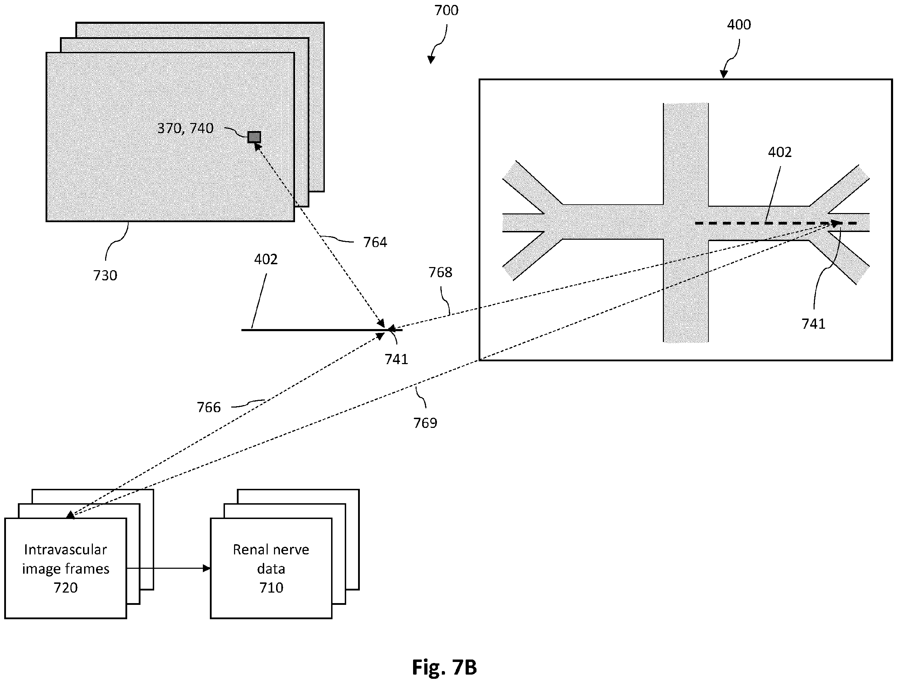

The plurality of nerve bundle data points is distinct from the plurality of intravascular images. Co-registration of the intravascular images themselves, rather than the nerve bundle data points, is shown in Figure 7B.

**Figure 7B** is a is a schematic, diagrammatic representation 700 of the co-registration of intravascular image frames 720 with the angiographic roadmap image 400 of the renal arterial tree, according to aspects of the present disclosure. In the example shown in Figure 7B, the location of the imaging element 370 or RDN treatment device 740 is visible in frames 730 of a live fluoroscopic X-ray display taken during the RDN procedure. Each such location is correlated with an intravascular image frame 720 along the pullback path 402, such that the intravascular image frames 720 can be shown (e.g., as visual annotations) at the locations of the intravascular image frames 720 on the pullback path 402 of the roadmap image 400, along with the current location of the imaging array 370 or RDN treatment device 740.

As shown by the arrow 764, because the pullback path 402 is correlated with the fluoroscopy images 730, each location 741 along the path 402 may be associated with one or more fluoroscopy images 730. In addition, because a correspondence was also established between the fluoroscopy images 730 and the intravascular image frames 720, the image frames 720 may also be associated with the location 741 along the path 402 as shown by the arrow 766. Just as different fluoroscopy images 730 and intravascular image frames 720 were associated with various locations along the pullback path 402, the fluoroscopy images 730 image frames 720 may be associated with the same locations along the roadmap image 400, as shown by the arrow 768 and the arrow 769.

In cases where the renal nerve images are derived from CT image data rather than intravascular data, the RDN nerve data co-registration system will determine a mapping between corresponding locations of the first three-dimensional (3D) model of the renal arterial tree based on the computed tomography (CT) imaging data, and a second 3D model of the blood vessel based on the X-ray angiography data.

Aspects of co-registration are described for example in U.S. Patent No. 7,930,014, titled "Vascular image co-registration", and U.S. Publication No. 2012/0004537, titled "Co-use of endoluminal data and extraluminal imaging", each of which is incorporated by reference as though fully set forth herein.

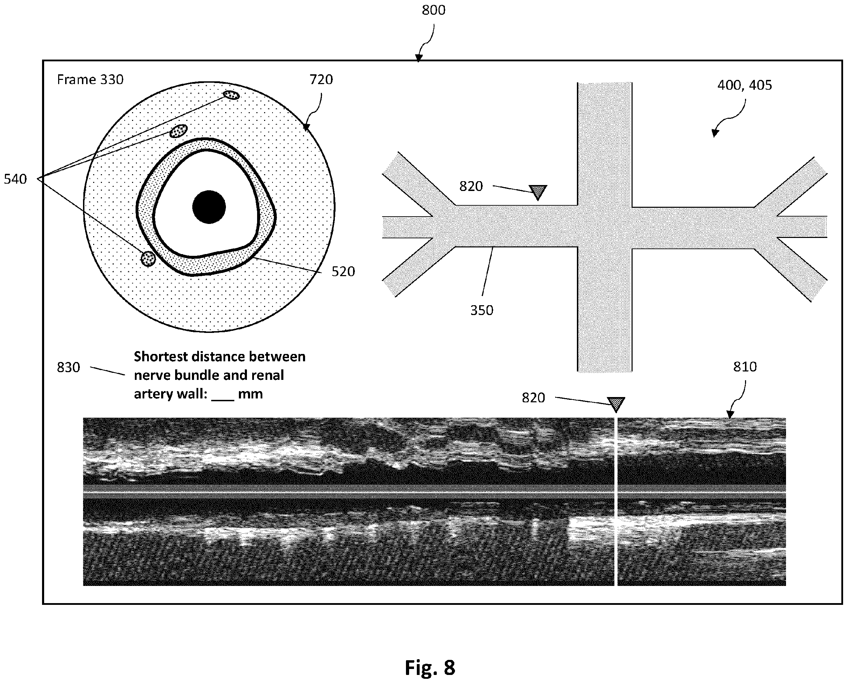

**Figure 8** is a co-registration screen display 800 of an example RDN nerve data co-registration system, according to aspects of the present disclosure. The co-registration screen display 800 includes a roadmap image 400 of the renal arterial tree 405, an image longitudinal display (ILD) 810, a location marker 820 in both the ILD 810 and the roadmap image 400, and a tomographic intravascular image 720 associated with the location of the location marker 820. In the example shown in Figure 8, the location marker 820 shows a location where a nerve bundle 540 is closest to the artery wall 520. Also visible is a text annotation 830 describing one or more characteristics of the location. This may for example include information about the renal nerve bundles adjacent to the wall 520 of the right renal artery 350. In some aspects, a location with the most favorable anatomy for RDN treatment may be identified and displayed automatically by the RDN nerve data co-registration system.

In other aspects, multiple locations or location ranges with favorable anatomy for RDN treatment may be identified and displayed automatically by the RDN nerve data co-registration system. Based on this information, the clinician can then apply human judgment to decide whether to perform RDN treatment at the identified location(s). In some aspects, the clinician can drag the location marker 820 along the renal artery 350 to see the intravascular images 720 and text annotations 830 associated with each location along the renal artery. In such aspects, the text annotations may be or include the renal nerve data points 710 of Figure 7.

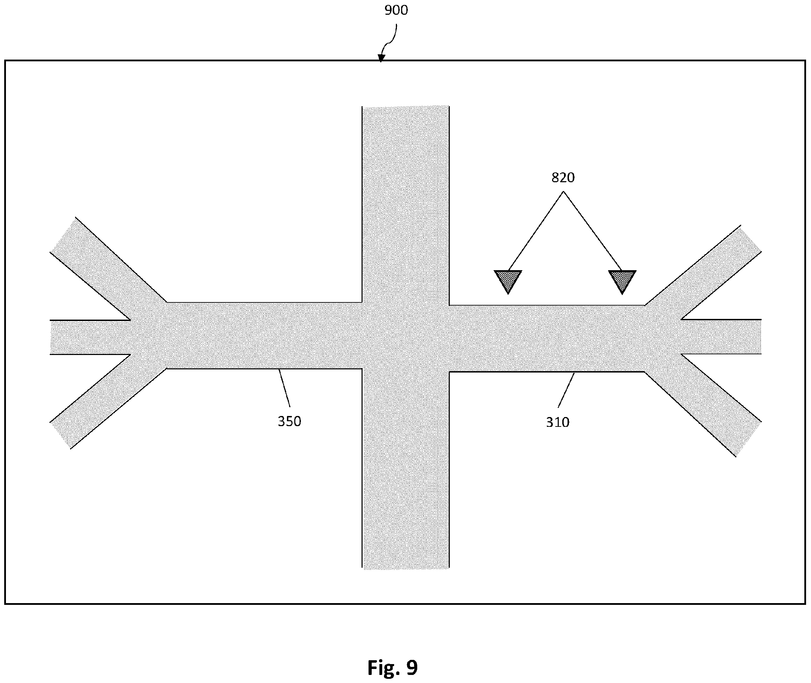

**Figure 9** is a roadmap image screen display 900 for an example RDN nerve data co-registration system, according to aspects of the present disclosure. In the example shown in Figure 9, location markers 820 have been used to mark two automatically identified locations along the left renal artery 310. These may for example be two locations where three nerve bundles fall within the depth of RDN treatment. In an example, one of the location markers 820 marks a location where two nerve bundles are extremely close to the artery wall (e.g., less than 1 mm from the artery wall), while the other location marker 820 marks a location where one nerve bundle is extremely close (e.g., less than 1 mm) to the artery wall. Since either of these locations could be favorable for RDN treatment, the RDN nerve data co-registration system has marked both of them on the screen display 900. The clinician may then decide to apply RDN treatment at either, both, or neither of the identified locations.

It is noted that, depending on the implementation, angiographic images of the left and right renal artery may be captured simultaneously, or may be captured at different times and later combined into a single roadmap screen display 900.

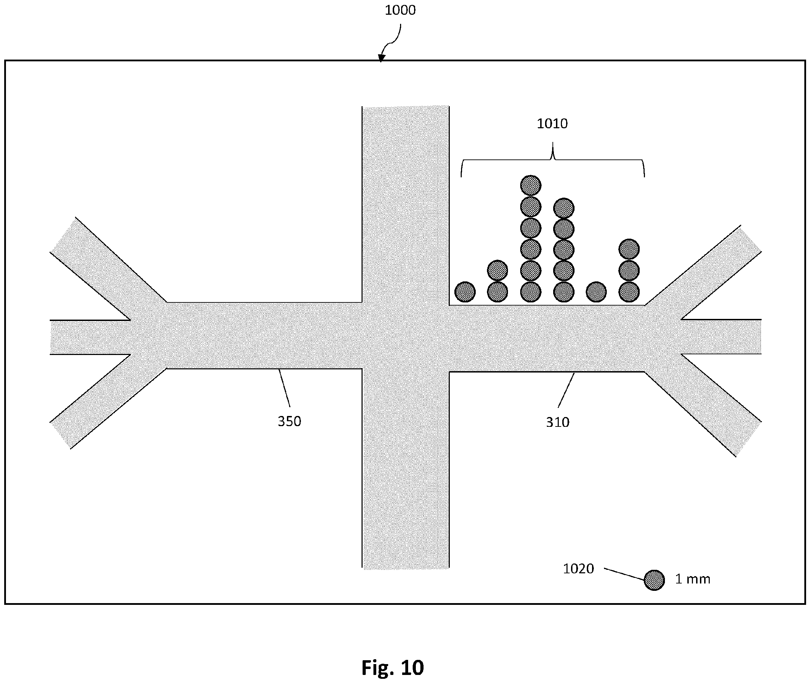

**Figure 10** is a roadmap image screen display 1000 for an example RDN nerve data co-registration system, according to aspects of the present disclosure. In the example shown in Figure 10, locations along the left renal artery 310 have been annotated with numerical displays 1010 showing a renal nerve metric or data point for each location. The metric or data point may for example be a distance from the artery wall to the nerve bundles (e.g., average, median, minimum, maximum, or weighted average distance). These numerical displays may for example be dots 1020, where each dot represents one millimeter of distance. This provides a visual cue to the clinician, that treatment may be more favorable in locations with one or two dots 1020 than in locations with multiple dots 1020. In some aspects, a location with zero dots may be indicative of one or more nerve bundles in direct contact with the artery wall, and a location marked with an "X" may indicate that no nerve bundles were identified at all in that location. In other aspects, the numerical indicators may be bars of varying length, Arabic numerals, or otherwise. A clinician can use this information in deciding at which location(s) along the length of the artery 310 to apply the RDN treatment.

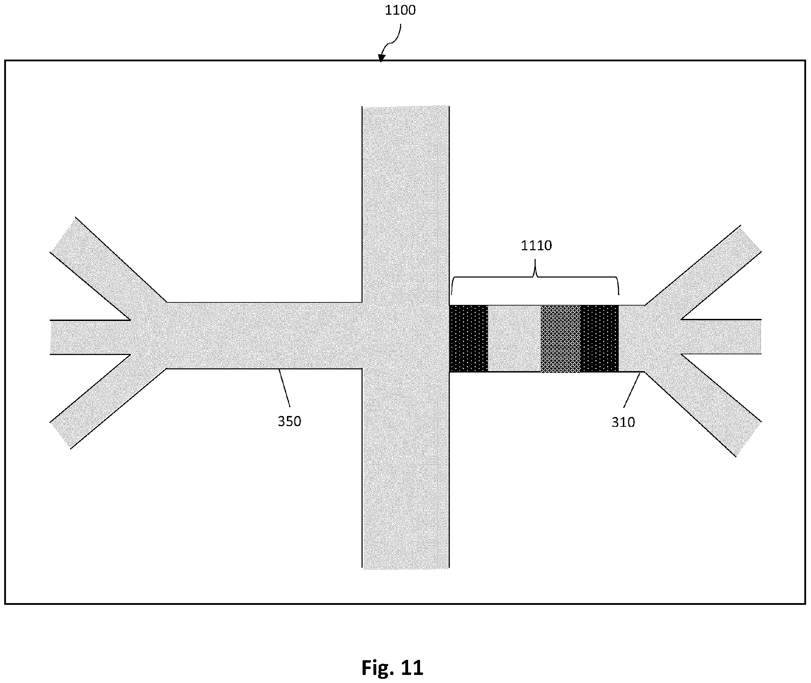

**Figure 11** is a roadmap image screen display 1100 for an example RDN nerve data co-registration system, according to aspects of the present disclosure. In the example shown in Figure 11, locations along the left renal artery 310 have been annotated with color-coded displays 1110 showing the metric or data point for each location along the renal artery 310. The metric or data point may for example be the distance from the artery wall to the nerve bundles (e.g., average, median, minimum, maximum, or weighted average distance). For example, a darker color may indicate areas where the nerve bundles are closer to the artery 310 (or where a larger number of nerve clusters are within the treatment penetration depth, etc.), while a lighter color may indicate areas where the nerve bundles are farther from the artery, not detected, etc. (or where a smaller number of nerve bundles, or zero nerve bundles, are within the treatment penetration depth). In other aspects, these quantities can be indicated by red and green coloring, respectively, or other combinations of hue, brightness, shading, etc. that effectively communicate the distance, nerve count, nerve density information, etc. to the clinician. The clinician can then use this information to decide which at location(s) along the length of the artery 310, if any, to apply the RDN treatment.

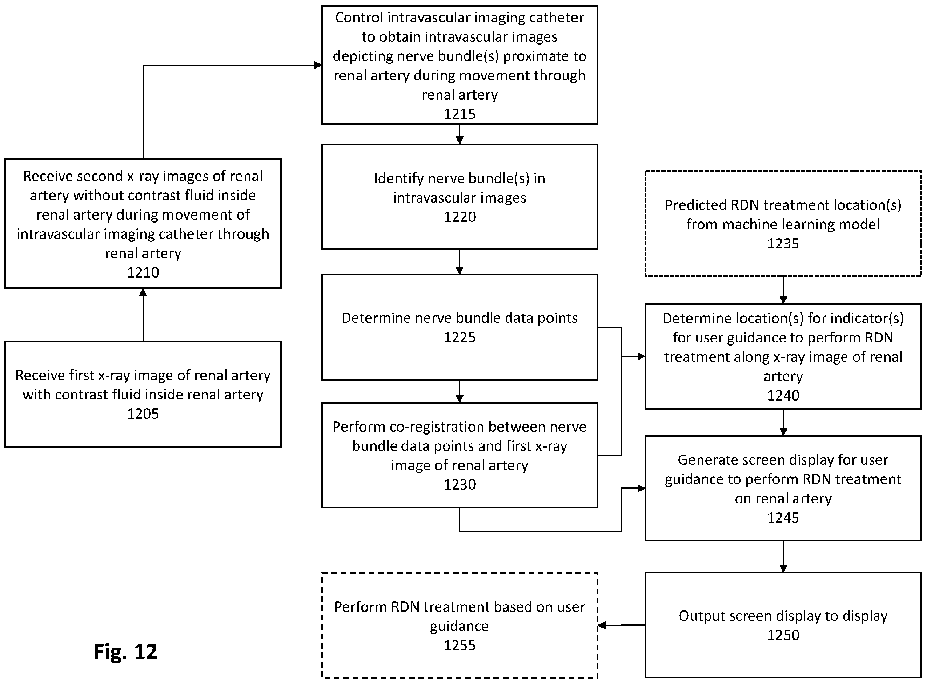

**Figure 12** is a schematic, diagrammatic representation, in flow diagram form, of an example RDN nerve data co-registration method 1200, according to aspects of the present disclosure. It is understood that the steps of method 1200 may be performed in a different order than shown in Figure 12, additional steps can be provided before, during, and after the steps, and/or some of the steps described can be replaced or eliminated in other embodiments. One or more of steps of the method 1200 can be carried by one or more devices and/or systems described herein, such as components of the system 100 and/or processor circuit 1050.

In step 1205, the method 1200 includes receiving a first X-ray image of the renal artery, with contrast fluid inside the renal artery, for use as a roadmap image. Execution then proceeds to step 1210.

In step 1210, the method 1200 includes receiving a second series of X-ray images of the renal artery, without contrast fluid inside the renal artery, during movement of an intravascular imaging catheter through the renal artery. Execution then proceeds to step 1215.

In step 1215, the method 1200 includes controlling the intravascular imaging catheter, during movement of the catheter through the renal artery, to obtain intravascular images of the renal artery, depicting nerve bundles proximate to the renal artery. Execution then proceeds to step 1220.

In step 1220, the method 1200 includes identifying the nerve bundles, if any, in the intravascular images. Execution then proceeds to step 1225.

In step 1225, the method 1200 includes determining the nerve bundle data points, as described above in Figures 6, 7A, and 8-11. Execution then proceeds to step 1230 and/or step 1240.

In step 1230, the method 1200 includes performing co-registration between the nerve bundle data points and the first X-ray image (e.g., the roadmap image) of the renal artery, as described above in Figure 7A. Execution then proceeds to step 1240 and/or step 1245.

In step 1240, the method 1200 optionally includes receiving one or more predicted RDN treatment locations 1235 from a trained machine learning model. In step 1240, the method 1200 also includes determining locations on the first X-ray image (e.g., the roadmap image) for the indicators that provide user guidance to perform RDN treatment. It is noted that co-registration and the machine learning algorithm could be used independently or together. One example includes displaying the co-registered IVUS metric adjacent to the angiogram, while also displaying the predicted RDN treatment locations from the machine learning algorithm. In some cases, the combination of the co-registration and the machine learning indicator(s) may guide the user to an optimal location or treatment zone.

Execution then proceeds to step 1245.

In step 1245, the method 1200 includes generating one or more screen displays to provide user guidance on where to perform the RDN treatment on the renal artery, as shown for example in Figures 8-11. Execution then proceeds to step 1250.

In step 1250, the method 1200 includes outputting the screen display to a video display (e.g., monitor 108 of Figure 1). Execution then optionally proceeds to step 1255.

In step 1255, the method 1200 includes, for the clinician, performing the RDN treatment based on the user guidance provided in the screen display. The method 1200 is now complete.

Flow diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information not otherwise available.

Similarly, the logic of flow diagrams may be shown as sequential. However, similar logic could be parallel, massively parallel, object oriented, real-time, event-driven, cellular automaton, or otherwise, while accomplishing the same or similar functions. In order to perform the methods described herein, a processor may divide each of the steps described herein into a plurality of machine instructions, and may execute these instructions at the rate of several hundred, several thousand, several million, or several billion per second, in a single processor or across a plurality of processors. Such rapid execution may be necessary in order to execute the method in real time or near-real time as described herein. For example, the identification of nerve bundles, calculation of nerve bundle data points, and co-registration of nerve bundle data points onto the roadmap image may occur in real time or near-real time.

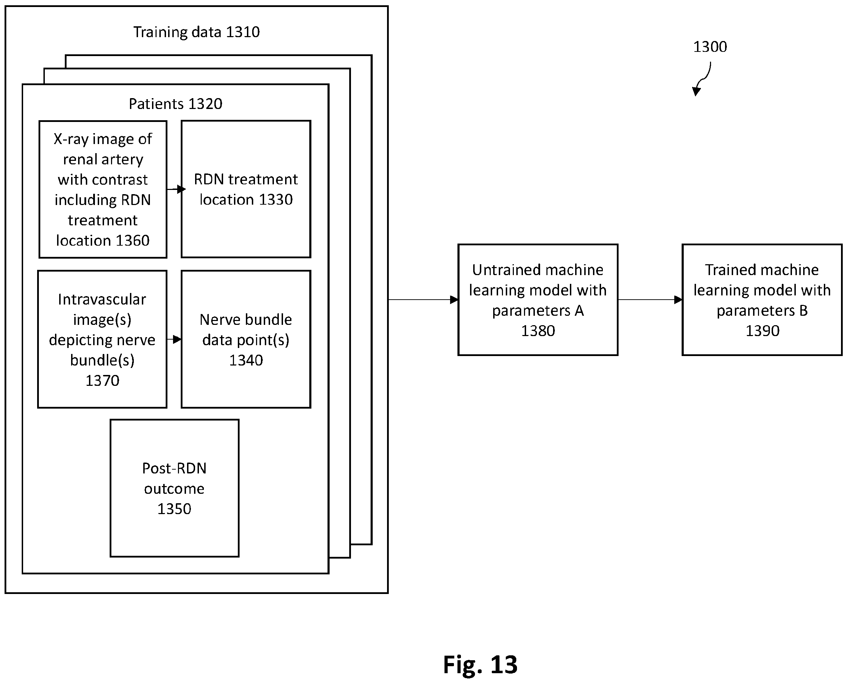

**Figure 13** is a schematic, diagrammatic representation, in block diagram form, of an example machine learning model training process 1300 for identifying RDN treatment locations, according to aspects of the present disclosure. The training process 1300 begins with a set of training data 1310 that includes data for a population of patients 1320. The data can for example include RDN treatment locations 1330, nerve bundle data points 1340, and post-RDN outcomes 1350 (e.g., an amount of reduction in systolic blood pressure 3 months or 6 months following the procedure) for each patient 1320. In other aspects, the data can include X-ray images with contrast (e.g., roadmap images) 1360 that are annotated with the RDN treatment locations 1330, as well as intravascular images 1370 depicting the nerve bundles, along with associated nerve bundle data points 1340.

These values 1330, 1340, 1350 (and potentially images 1360, 1370) are used to train an untrained machine learning model 1380 with parameters A, to produce a trained machine learning model 1390 with parameters B, that can be used in inference mode to calculate treatment locations for a new patient. In one aspect, the machine learning model is a supervised learning model based on multivariate regression models. In another aspect, the machine learning model is based on trained object detection in computer vision techniques; convolutional neural networks such as AlexNet, Fast RCNN, and Faster RCNN, or one-step architectures such as YOLO, SSD, and RetinaNet.

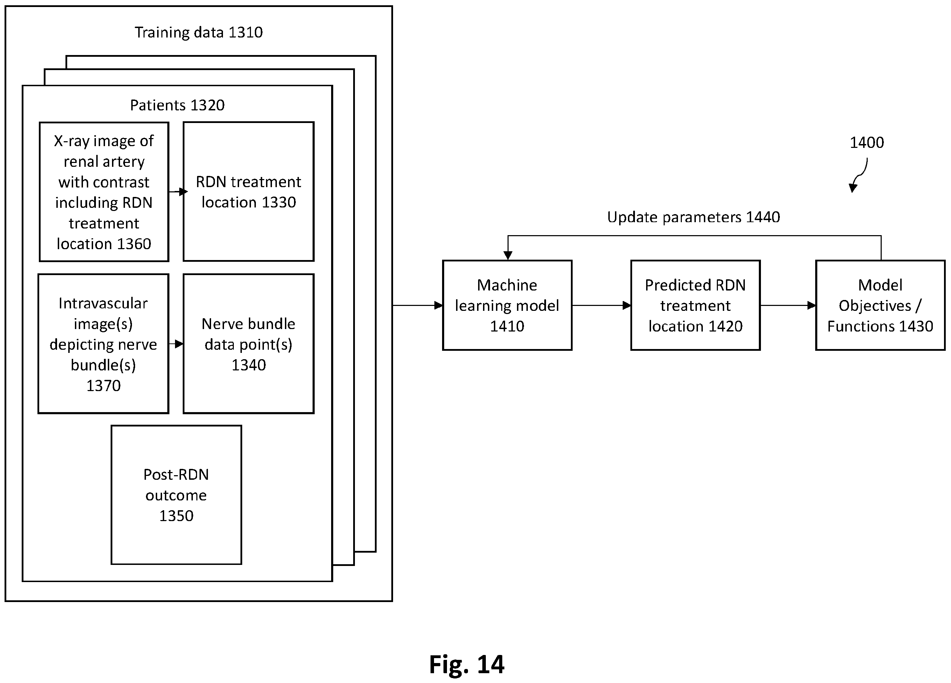

**Figure 14** is a schematic, diagrammatic representation, in block diagram form, of an example machine learning model training system 1400 for RDN treatment location calculation, according to aspects of the present disclosure. The training system 1400 begins with a set of training data 1310 that includes data for a population of patients 1320. The data can for example include RDN treatment locations 1330, nerve bundle data points 1340, and post-RDN outcomes 1350 (e.g., an amount of reduction in systolic blood pressure 3 months or 6 months post-procedure) for each patient 1320. In other aspects, the data can include X-ray images with contrast (e.g., roadmap images) 1360 that are annotated with the RDN treatment locations 1330, as well as intravascular images 1370 depicting the nerve bundles, along with associated nerve bundle data points 1340.

These values 1330, 1340, 1350 (and potentially images 1360, 1370) are used to train a predictive machine learning network or model 1410, which produces predicted RDN treatment locations 1420 for each patient 1320. These locations 1420 are then evaluated against the model's objectives or functions 1430 (e.g., a difference between the RDN treatment location 1330 and the predicted RDN treatment location 1420, a difference between the post-RDN outcome 1350 and the expected post-RDN outcome associated with the predicted RDN treatment location 1420) to determine the success of the training, and, if the predicted RDN locations 1420 fall below a threshold of desired accuracy (e.g. for predicting the post-RDN outcomes 1350), the parameters go through repeated updates 1440 until the desired accuracy is achieved. In some instances, the updating 1440 may be accomplished using gradients of the objective functions and backpropagation to update the parameters of the predictive network 1410. In some embodiments, retraining and/or fine tuning can be done for newly introduced patient data (e.g., within the context of a clinical trial).

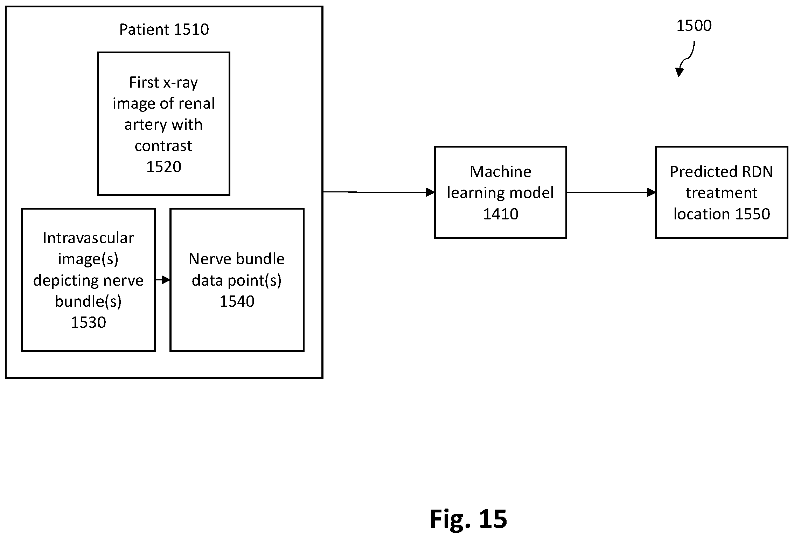

**Figure 15** is a schematic, diagrammatic representation, in block diagram form, of an example machine learning model inference mode 1500 for RDN treatment location calculation, according to aspects of the present disclosure. The trained machine learning model 1410 receives data for a new patient 1510, which may for example include the first X-ray image 1520 of the renal artery with contrast (e.g., the roadmap image), the intravascular images 1530 depicting the nerve bundles, and/or the calculated nerve bundle data points 1540 for the new patient 1510. From this input data, the trained machine learning model 1410 produces one or more RDN treatment locations 1550, where treatment is determined by the machine learning model 1410 to be likely to produce a favorable treatment outcome (e.g., a reduction in systolic blood pressure of 5mmHg or greater). This may for example provide a clinician with the information needed to determine whether to perform an RDN procedure on the patient, and if so, what location(s) in the renal artery to perform the treatment.

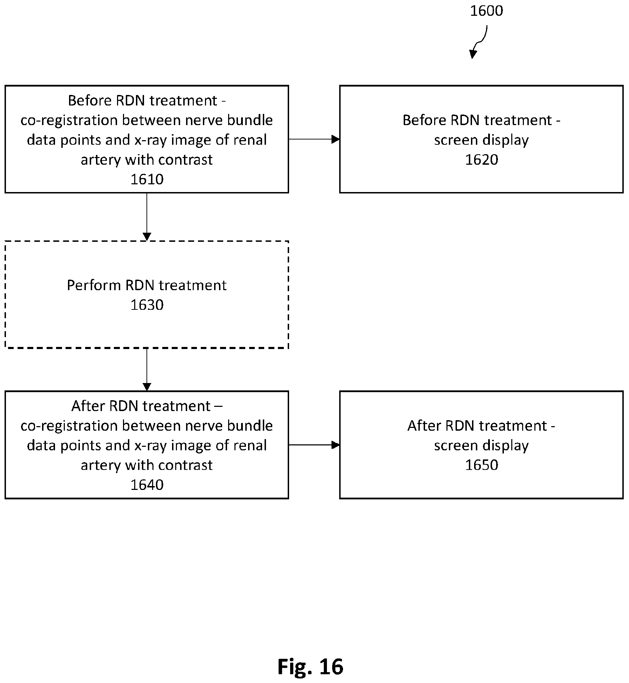

**Figure 16** is a schematic, diagrammatic representation, in flow diagram form, of an example RDN treatment evaluation process 1600, according to aspects of the present disclosure.

In step 1610, the method 1600 includes, before the RDN treatment has taken place, performing the co-registration between the nerve bundle data points and the X-ray image of the renal artery with contrast (e.g., the roadmap image), and generating/displaying a pre-RDN-treatment screen display 1620.

In step 1630, the method 1600 includes the clinician performing the RDN treatment.

In step 1640, the method 1600 includes co-registration between post-treatment nerve bundle data points and the X-ray image of the renal artery with contrast (e.g., the roadmap image).

In some aspects, the post-treatment nerve bundle data points are calculated the same way as the pre-treatment nerve bundle data points. The effects of ablation change material properties of the tissue, which allows the computer vision system to detect the presence of ablated and healthy nerves in the same frame, or in a comparison between the pre- and post-treatment images. In such cases, the clinician may for example check to see that there are no longer any healthy nerve fiber bundles, in any location along the renal artery, that are within the RDN treatment depth, and of not, the clinician may deem the treatment to be successful.

In other aspects, the post-treatment nerve bundle data points may for example be or include a level of ablation of the nerve fibers. In such cases, the clinician may for example check to see that all of the selected treatment locations show 50% or greater ablation of the nerve fiber bundles within the RDN treatment depth, and if so, may deem the treatment to be successful.

Still other success metrics may be used instead or in addition, including but not limited to area and or volume of ablation within the post-treatment imaging data, percentage of thermal ablation within the treatment penetration depth, ratio of thermal ablation volume or area and the theoretical treatment volume or area, volume or area of ablated nerve bundles, percentage of ablated nerve bundles, or percentage of ablation per each nerve bundle.

In step 1640, the method 1600 also includes generating and displaying a post-treatment screen display 1650.

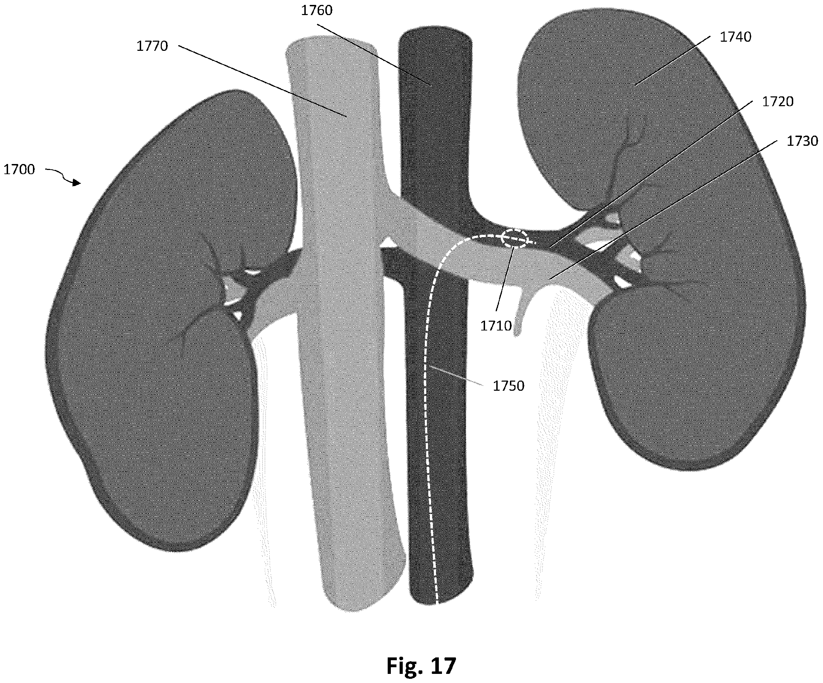

**Figure 17** is a schematic, diagrammatic representation of the renal vasculature 1700 of a patient, with a renal denervation treatment device 1710 in the left renal artery 1720 of the left kidney 1740, according to aspects of the present disclosure. In an example, the renal denervation treatment device 1710 may include a catheter 1750 with a renal denervation tool (e.g., electrodes, balloon, etc.) that delivers energy to injure the renal nerves, in order to lower the patient's blood pressure. Depending on the implementation, the delivered energy may be electrical energy, chemical energy, heat, cold, etc. The renal denervation treatment device 1710 may for example enter the renal artery 1720 via the abdominal aorta 1760. Also visible is the renal vein 1730.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes an apparatus that includes a processor circuit configured for communication with an intravascular imaging catheter, where the processor circuit is configured to: control the intravascular imaging catheter to obtain a plurality of intravascular images during movement of the intravascular imaging catheter along a length of a renal artery, where the plurality of intravascular images depicts one or more nerve bundles for targeting during a renal denervation treatment; perform co-registration between: a plurality of nerve bundle data points representative of the one or more nerve bundles and associated with a treatment location along the length of the renal artery for the renal denervation treatment; and a first X-ray image of the renal artery obtained with a contrast fluid inside the renal artery; and output, to a display, a screen display configured to guide a user regarding the renal denervation treatment, where the screen display is based on the co-registration. Other embodiments of this aspect include corresponding computer systems, apparatus, and computer programs recorded on one or more computer storage devices, each configured to perform the actions of the methods.

Implementations may include one or more of the following features. In some aspects, the screen display may include the first X-ray image overlaid with a plurality of representations of the plurality of nerve bundle data points, where each representation of the plurality of representations is co-located with a data point of the plurality of nerve bundle data points. In some aspects, each representation may include a number of dots proportional to a numerical value of a corresponding data point of the plurality of nerve bundle data points. In some aspects, the processor circuit is configured to perform co-registration between the plurality of intravascular images and the first X-ray image separate from the co-registration between the plurality of nerve bundle data points and the first x-ray image of the renal artery. In some aspects, each representation may include a color or shading corresponding to a numerical value of a corresponding data point of the plurality of nerve bundle data points. In some aspects, the display further may include a location marker indicative of a location along the renal artery. In some aspects, the screen display further may include a tomographic image of the renal artery at the location indicated by the location marker. In some aspects, the tomographic image may include nerve bundles corresponding to a nerve bundle data point for the location indicated by the location marker. In some aspects, the location indicated by the location marker is a treatment location selected by a trained machine-learning model. In some aspects, the screen display further may include an image longitudinal display, where the location marker appears on both the image longitudinal display and the first X-ray image. In some aspects, after the co-registration between the plurality of nerve bundle data points and the first X-ray image is performed, the plurality of nerve bundle data points is respectively associated with a plurality of locations along the length of the renal artery. In some aspects, the plurality of nerve bundle data points may include at least one of: a distance between a border of the renal artery and the one or more nerve bundles; a quantity of the one or more nerve bundles; a size of the one or more nerve bundles; or a quantity of the one or more nerve bundles located within a penetration depth of a renal denervation treatment device used to perform the renal denervation treatment. In some aspects, the plurality of nerve bundle data points is distinct from the plurality of intravascular images. In some aspects, to perform the co-registration between the plurality of nerve bundle data points and the first X-ray image of the renal artery, the processor circuit is configured to: receive the first X-ray image of the renal artery; receive a plurality of second x-ray images of the renal artery during the movement of the intravascular imaging catheter, where the plurality of second X-ray images is obtained without the contrast fluid inside the renal artery and depicts a plurality of locations of the intravascular imaging catheter during the movement; and perform the co-registration between the plurality of nerve bundle data points and the first X-ray image of the renal artery using the plurality of second X-ray images. Implementations of the described techniques may include hardware, a method or process, or computer software on a computer-accessible medium.

One general aspect includes a method. The method includes controlling, with a processor circuit, an intravascular imaging catheter in communication with the processor circuit to obtain a plurality of intravascular images during movement of the intravascular imaging catheter along a length of a renal artery, where the plurality of intravascular images depicts one or more nerve bundles for targeting during a renal denervation treatment; performing, with the processor circuit, co-registration between: a plurality of nerve bundle data points representative of the one or more nerve bundles and associated with a treatment location along the length of the renal artery for the renal denervation treatment; and an X-ray image of the renal artery obtained with a contrast fluid inside the renal artery; and outputting, to a display in communication with the processor circuit, a screen display configured to guide a user regarding the renal denervation treatment, where the screen display is based on the co-registration. Other embodiments of this aspect include corresponding computer systems, apparatus, and computer programs recorded on one or more computer storage devices, each configured to perform the actions of the methods.

Accordingly, it can be seen that the RDN nerve data co-registration system advantageously provides a means for determining, in real time during a procedure, the locations within the left or right renal artery that are most favorable for RDN treatment. The RDN nerve data co-registration system also provides a means to determine whether an RDN procedure is complete (e.g., whether the renal nerve bundles have been ablated sufficiently in the desired locations. The clinician may use this screen display 1650 to determine whether the RDN treatment is complete, or whether further treatment is needed to adequately ablate the renal nerve fiber bundles in the desired locations.

A number of variations are possible on the examples and aspects described above. For example, other variables may be used than those listed herein, and other sensors or sensor types may be employed. The technology described herein may be used not only before and during medical interventions, but also at other times, to provide metrics that may be indicative of a health state or disease state of the patient..

Accordingly, the logical operations making up the aspects of the technology described herein are referred to variously as operations, steps, objects, elements, components, or modules. Furthermore, it should be understood that these may occur, or be performed or arranged, in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the RDN nerve data co-registration system . Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the RDN nerve data co-registration system as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

Additional embodiments.
Embodiment 1. An apparatus, comprising:
   a processor circuit configured for communication with an intravascular imaging catheter, wherein the processor circuit is configured to:
   control the intravascular imaging catheter to obtain a plurality of intravascular images during movement of the intravascular imaging catheter along a length of a renal artery, wherein the plurality of intravascular images depicts one or more nerve bundles for targeting during a renal denervation treatment;
   perform co-registration between:
      a plurality of nerve bundle data points representative of the one or more nerve bundles and associated with a treatment location along the length of the renal artery for the renal denervation treatment; and
      a first X-ray image of the renal artery obtained with a contrast fluid inside the renal artery; and
   output, to a display, a screen display configured to guide a user regarding the renal denervation treatment, wherein the screen display is based on the co-registration.
Embodiment 2. The apparatus of Embodiment 1, wherein the screen display comprises the first X-ray image overlaid with a plurality of representations of the plurality of nerve bundle data points, wherein each representation of the plurality of representations is co-located with a data point of the plurality of nerve bundle data points.
Embodiment 3. The apparatus of Embodiment 2, wherein each representation comprises a number of dots proportional to a numerical value of a corresponding data point of the plurality of nerve bundle data points.
Embodiment 4. The apparatus of Embodiment 2, wherein each representation comprises a color or shading corresponding to a numerical value of a corresponding data point of the plurality of nerve bundle data points.
Embodiment 5. The apparatus of Embodiment 2, wherein the display further comprises a location marker indicative of a location along the renal artery.
Embodiment 6. The apparatus of Embodiment 5, wherein the screen display further comprises a tomographic image of the renal artery at the location indicated by the location marker.
Embodiment 7. The apparatus of Embodiment 6, wherein the tomographic image comprises nerve bundles corresponding to a nerve bundle data point for the location indicated by the location marker.
Embodiment 8. The apparatus of Embodiment 5, wherein the location indicated by the location marker is a treatment location selected by a trained machine-learning model.
Embodiment 9. The apparatus of Embodiment 5, wherein the screen display further comprises an image longitudinal display, and wherein the location marker appears on both the image longitudinal display and the first X-ray image.
Embodiment 10. The apparatus of Embodiment 1, wherein, after the co-registration between the plurality of nerve bundle data points and the first X-ray image is performed, the plurality of nerve bundle data points is respectively associated with a plurality of locations along the length of the renal artery.
Embodiment 11. The apparatus of Embodiment 1, wherein the plurality of nerve bundle data points comprises at least one of:
   a distance between a border of the renal artery and the one or more nerve bundles;
   a quantity of the one or more nerve bundles;
   a size of the one or more nerve bundles; or
   a quantity of the one or more nerve bundles located within a penetration depth of a renal denervation treatment device used to perform the renal denervation treatment.
Embodiment 12. The apparatus of Embodiment 1, wherein the plurality of nerve bundle data points is distinct from the plurality of intravascular images.
Embodiment 13. The apparatus of Embodiment 3, wherein the processor circuit is configured to perform co-registration between the plurality of intravascular images and the first X-ray image separate from the co-registration between the plurality of nerve bundle data points and the first X-ray image of the renal artery.
Embodiment 14. The apparatus of Embodiment 1, wherein, to perform the co-registration between the plurality of nerve bundle data points and the first X-ray image of the renal artery, the processor circuit is configured to:
   receive the first X-ray image of the renal artery;
   receive a plurality of second X-ray images of the renal artery during the movement of the intravascular imaging catheter, wherein the plurality of second X-ray images is obtained without the contrast fluid inside the renal artery and depicts a plurality of locations of the intravascular imaging catheter during the movement; and
   perform the co-registration between the plurality of nerve bundle data points and the first X-ray image of the renal artery using the plurality of second X-ray images.
Embodiment 15. A method, comprising:
   controlling, with a processor circuit, an intravascular imaging catheter in communication with the processor circuit to obtain a plurality of intravascular images during movement of the intravascular imaging catheter along a length of a renal artery, wherein the plurality of intravascular images depicts one or more nerve bundles for targeting during a renal denervation treatment;
   performing, with the processor circuit, co-registration between:
      a plurality of nerve bundle data points representative of the one or more nerve bundles and associated with a treatment location along the length of the renal artery for the renal denervation treatment; and
      an X-ray image of the renal artery obtained with a contrast fluid inside the renal artery; and
   outputting, to a display in communication with the processor circuit, a screen display configured to guide a user regarding the renal denervation treatment, wherein the screen display is based on the co-registration.
Embodiment 16. A method, comprising:
   performing, with a processor circuit, co-registration between:
      a plurality of nerve bundle data points representative of one or more nerve bundles for targeting during a renal denervation treatment and associated with a treatment location along a length of a renal artery for the renal denervation treatment, the one or more nerve bundles being depicted within a plurality of intravascular images obtained by moving the intravascular imaging catheter along the length of the renal artery; and
      an X-ray image of the renal artery obtained with a contrast fluid inside the renal artery; and
   outputting, to a display in communication with the processor circuit, a screen display configured to guide a user regarding the renal denervation treatment, wherein the screen display is based on the co-registration; and
   optionally, controlling the display to output the screen display to the user.
Embodiment 17. The method of claim 16, further comprising performing with the processor circuit, controlling, with a processor circuit, an intravascular imaging catheter in communication with the processor circuit to obtain a plurality of intravascular images during movement of the intravascular imaging catheter along a length of a renal artery, wherein the plurality of intravascular images depicts one or more nerve bundles for targeting during a renal denervation treatment.
Embodiment 18. Computer program comprising instructions which, when executed, cause the implementation of the method of embodiment 16 or 17.
Embodiment 19. Computer program comprising instructions which, when executed, causes the execution of the computer program of embodiment 18.
Embodiment 20. Computer program according to embodiment 19 which, when executed, causes the controlling of the display to output the screen display to the user; and, optionally, causes the outputting of the screen display to the display.

## Claims

1. A processor circuit configured for communication with an intravascular imaging catheter, wherein the processor circuit is configured to:
perform co-registration between:
a plurality of nerve bundle data points representative of one or more nerve bundles for targeting during a renal denervation treatment and associated with a treatment location along a length of a renal artery for renal denervation treatment, the one or more nerve bundles being depicted within a plurality of intravascular images obtained by moving the intravascular imaging catheter along the length of the renal artery; and
a first X-ray image of the renal artery obtained with a contrast fluid inside the renal artery; and
output, to a display, a screen display configured to guide a user regarding the renal denervation treatment, wherein the screen display is based on the co-registration.

2. The processor circuit of claim 1, wherein the screen display comprises the first X-ray image overlaid with a plurality of representations of the plurality of nerve bundle data points, wherein each representation of the plurality of representations is co-located with a data point of the plurality of nerve bundle data points.

3. The processor circuit of claim 2, wherein each representation of the plurality of representations comprises a number of dots proportional to a numerical value of a corresponding data point of the plurality of nerve bundle data points; or
wherein each representation of the plurality of representation comprises a color or shading corresponding to a numerical value of a corresponding data point of the plurality of nerve bundle data points.

4. The processor circuit of any one of the previous claims, wherein the screen display further comprises a location marker indicative of a location along the renal artery.

5. The processor circuit of claim 4, wherein the screen display further comprises a tomographic image of the renal artery at the location indicated by the location marker, , wherein the tomographic image comprises nerve bundles corresponding to a nerve bundle data point for the location indicated by the location marker.

6. The processor circuit of claim 4, wherein the location indicated by the location marker is a treatment location selected by a trained machine-learning model.

7. The processor circuit of claim 4, wherein the screen display further comprises an image longitudinal display, and wherein the location marker appears on both the image longitudinal display and the first X-ray image.

8. The processor circuit of any one of the previous claims, wherein, after the co-registration between the plurality of nerve bundle data points and the first X-ray image is performed, the plurality of nerve bundle data points is respectively associated with a plurality of locations along the length of the renal artery.

9. The processor circuit of any one of the previous claims, wherein the plurality of nerve bundle data points comprises at least one of:
a distance between a border of the renal artery and the one or more nerve bundles;
a quantity of the one or more nerve bundles;
a size of the one or more nerve bundles; or
a quantity of the one or more nerve bundles located within a penetration depth of a renal denervation treatment device used to perform the renal denervation treatment.

10. The processor circuit of any one of the previous claims, wherein the plurality of nerve bundle data points is distinct from the plurality of intravascular images.

11. The processor circuit of claim 3, wherein the processor circuit is configured to perform co-registration between the plurality of intravascular images and the first X-ray image separate from the co-registration between the plurality of nerve bundle data points and the first X-ray image of the renal artery.

12. The processor circuit of any one of the previous claims, wherein, to perform the co-registration between the plurality of nerve bundle data points and the first X-ray image of the renal artery, the processor circuit is configured to:
receive the first X-ray image of the renal artery;
receive a plurality of second X-ray images of the renal artery during the movement of the intravascular imaging catheter, wherein the plurality of second X-ray images is obtained without the contrast fluid inside the renal artery and depicts a plurality of locations of the intravascular imaging catheter during the movement; and
perform the co-registration between the plurality of nerve bundle data points and the first X-ray image of the renal artery using the plurality of second X-ray images.

13. An apparatus comprising a processor circuit according to any one of the claims 1 to 12, the apparatus comprising at least one of a display device for communicating with the processor circuit or the intravascular imaging catheter.

14. A method, comprising:
performing, with a processor circuit, co-registration between:
a plurality of nerve bundle data points representative of one or more nerve bundles for targeting during a renal denervation treatment and associated with a treatment location along a length of a renal artery for the renal denervation treatment, the one or more nerve bundles being depicted within a plurality of intravascular images obtained by moving the intravascular imaging catheter along the length of the renal artery; and
an X-ray image of the renal artery obtained with a contrast fluid inside the renal artery; and
outputting, to a display in communication with the processor circuit, a screen display configured to guide a user regarding the renal denervation treatment, wherein the screen display is based on the co-registration; and
optionally, controlling the display to output the screen display to the user.

15. Computer program comprising instructions which, when executed, causes the execution of a computer program comprising instructions which, when executed, cause the implementation of the method of claim 14.
